# EUROPEAN PATENT SPECIFICATION

(11) **EP 2 276 797 B1**
(45) Date of publication and mention of the grant of the patent: **21.11.2012**
(21) Application number: 09742009.5
(22) Date of filing: 29.04.2009
(51) Int. Cl.: C08J 3/12, C08L 25/14, C08F 2/24, C09B 67/00

(54) **POLYMER ENCAPSULATED COLOURANTS BY SPRAY DRYING**
POLYMERGEKAPSELTE FÄRBEMITTEL MIT SPRÜHTROCKNUNG
COLORANTS À BASE DE POLYMÈRES ENCAPSULÉS PAR SÉCHAGE PAR PULVÉRISATION

(30) Priority: 09.05.2008 EP 08155935
(43) Date of publication of application: 26.01.2011
(73) Proprietor: BASF SE, 67056 Ludwigshafen (DE)
(72) Inventor: MISTRY, Kishor, Kumar, Bradford, West Yorkshire BD14 6LR (GB); BAXTER, Mark, Christopher, Bradford, West Yorkshire BD8 9EY (GB)
(86) International application number: PCT/EP2009/055188
(87) International publication number: WO 2009/135791

(56) References cited:
- WO-A-02/090445
- WO-A-2005/123796
- US-A- 4 892 932
- US-A- 5 462 978
- US-A1- 2005 276 774
- US-B1- 6 444 729

## Description

The invention relates to an improved process for the preparation polymer particles which comprise an effective amount of at least one colourant, such as pigments, dyes or lakes.

*WO 2005*/*123009* and *WO 123796* disclose a process for the preparation of polymer particles that contain within their polymer structure at least one colourant. These colour encapsulated polymers are obtained from water-in-oil emulsions. The process utilizes large amounts of hydrocarbon solvents and stabilizers. The hydrocarbon solvents have to be removed from the emulsion in a subsequent process step.

An objective of the present invention is to provide a technically more feasible and more convenient process which is useful for the preparation of personal care and/or cosmetic compositions. These compositions comprise polymeric particles containing entrapped or encapsulated colourants and retain the colourant over extended periods of time and also when subjected to different environments. This is especially important when the colourants are particularly water-soluble dyes, where it is generally difficult to permanently retain the dye. In a cosmetic composition, if the dye is not permanently retained, this can impair the visual effect of the cosmetic after prolonged use.

It has surprisingly been found that spray drying an aqueous emulsion that contains no hydrocarbon solvents is an alternative approach for the preparation of polymer particles that contain within their polymer structure at least one colourant.

The invention relates to an improved process of preparing polymer particles which comprise an effective amount of at least one colourant, a polymer matrix and secondary particles distributed within the polymer matrix,
wherein the polymer matrix is formed from a blend of monomers comprising a first monomer, which is an ethylenically unsaturated ionic monomer, and a second monomer, which is an ethylenically unsaturated hydrophobic monomer and which is capable of forming a homopolymer of glass transition temperature above 50°C as determined by Differential Scanning Calorimetry (DSC),
wherein the secondary particles comprise a hydrophobic polymer which is formed from an ethylenically unsaturated hydrophobic monomer which is capable of forming a polymer of glass transition temperature above 50°C and optionally other monomers, and wherein the hydrophobic polymer is different from the polymeric matrix,
which process essentially consists of the steps:
A) Preparing an emulsion of a polymer formed from a monomer blend in an aqueous phase which comprises the first and second monomers, forming the secondary particles in the aqueous phase or combining the secondary particles with the aqueous phase;
B) Adding to the aqueous phase an effective colouring amount of at least one colourant;
C) Dispersing the colourant and homogenising the aqueous phase; and
D) Subjecting the emulsion to dehydration in a spray-drying unit.

The invention further relates to a process for the preparation of personal care or cosmetic compositions, which comprises further processing to personal care or cosmetic compositions the polymer particles obtainable by the process defined above.

The micro-particulate colourant blends according to the invention have enhanced visual performance, such as a more natural skin like appearance. Furthermore the matrix polymer does not shatter under rigorous formulation conditions or handling, thus retaining the desirable aesthetic effects during storage and use.

Preferably the first monomer used to form the matrix polymer is present in the form of the salt of a volatile component forming a counter-ion. During the dehydration step D) that volatile component of the salt is evaporated. By this is meant that at least a part of the counterion component is evaporated. For instance, in the event that the polymer matrix is formed by the ammonium salt of monomer units, the volatile component ammonia will be evaporated in the dehydration step. During the distillation stage the matrix polymer will then be converted to its free acid form.

The particles useful in the invention comprise a colourant. The colourant may be selected from pigments, dyes or lakes. In the process of preparing the particles it is particularly desirable for the colourant to be dissolved or dispersed in the aqueous phase so that it can become distributed throughout the matrix polymer.

The polymeric microparticles described above exhibit improved shatter resistance in combination with improved visual performance. Furthermore, the polymer matrix does not allow the entrapped colourant to be released even under prolonged use. It is particularly desirable to provide particles in which the colourant is distributed throughout the matrix polymer and wherein the matrix polymer is impermeable to the colourant.

The rigidity of the polymer matrix can be further enhanced if the matrix polymer is crosslinked. This can be effected by including a cross-linking step in the process. This can be achieved by including self cross-linking groups in the polymer, for instance monomer repeating units carrying a methylol functionality. Preferably, the cross-linking is achieved by including a cross-linking agent with the aqueous phase polymer. Suitable cross-linking agents are compounds which react with functional groups on the polymer chain. For instance, when the polymer chain contains anionic groups, suitable cross-linking agents include aziridines, diepoxides, carbodiamides, silanes or multivalent metals, for instance aluminum, zinc or zirconium. A particularly preferred cross-linking agent is ammonium zirconium carbonate or zinc oxide. Another particularly preferred class of cross-linking agents includes compounds that form covalent bonds between polymer chains, for instance silanes or diepoxides. The cross-linking process desirably occurs during the dehydration step.

It has been found that polymers formed from the special combination of a hydrophobic monomer that is capable of forming a homopolymer of glass transition temperature in excess of 50°C, preferably greater than 60 or 80°C, exhibit considerably improved performance in regard to the impermeability to the colourant as well as other actives. By hydrophobic monomer is meant that the monomer has a solubility in water of less than 5 g per 100 ml of water.

The glass transition temperature (Tg) for a polymer is defined in the Kirk-Othmer, Encyclopedia of Chemical Technology, Volume 19, fourth edition, page 891, as the temperature below which (1) the transitional motion of entire molecules and (2) the coiling and uncoiling of 40 to 50 carbon atom segments of chains are both frozen. Thus, below its Tg a polymer would not exhibit flow or rubber elasticity. The Tg of a polymer may be determined using Differential Scanning Calorimetry (DSC).

Generally the average particle size diameter of the particles is less than about 100 µm (microns, micrometer). Preferably the average particle size diameter is in the range of about 1 to 60 µm, e.g. 1 to 40 µm and especially between 1 and 30 µm. The average particle size is determined by a Sympatec HELOS particle size analyzer according to standard procedures well documented in the literature.

The monomer blend in for producing the matrix polymer may contain at least 50% by weight hydrophobic monomer, the remainder being made up of ionic monomer. According to a preferred embodiment, the hydrophobic monomer will be present in amounts of at least 60% by weight. Preferred compositions contain between 65 and 90% by weight hydrophobic polymer, for instance around 70 or 75%.

Specific examples of hydrophobic monomers include styrene, C₁-C₄alkyl(meth)acrylate, such as methyl methacrylate, methyl acrylate, ethyl (meth)acrylate or n- or tertiary butyl (meth)-acrylate, phenyl methacrylate, C₅-C₁₂cycloalkyl(meth) acrylate, such as cyclohexyl methacrylate or isobornyl methacrylate.

According to a preferred embodiment, the Tg should be above 60°C or above 80°C.

The ionic monomer may contain either anionic or cationic groups, or alternatively may be potentially ionic, for instance in the form of an acid anhydride. Preferably the ionic monomer is an ethylenically unsaturated anionic or potentially anionic monomer. Suitable anionic or potentially anionic monomers include acrylic acid, methacrylic acid, ethyl acrylic acid, fumaric acid, maleic acid, maleic anhydride, itaconic acid, itaconic acid anhydride, crotonic acid, vinyl acetic acid, (meth)allyl sulphonic acid, vinyl sulphonic acid and 2-acrylamido-2-methyl propane sulphonic acid. Preferred anionic monomers are carboxylic acids or acid anhydrides, such as (meth)acrylic acid.

When the ionic monomer is anionic, for instance a carboxylic acid or anhydride, the volatile counterion may be ammonia or a volatile amine component. The volatile amine component is a liquid that can be evaporated at low to moderate temperatures, for instance by temperatures up to 200°C. Preferably, it will be possible to evaporate the volatile amine under reduced pressure at temperatures below 100 °C. The polymer may be produced in free acid form and then neutralized with an aqueous solution of ammonium hydroxide or a volatile amine, for instance ethanolamine, methanolamine, 1-propanolamine, 2-propanolamine, dimethanol-amine or diethanolamine. Alternatively the polymer may be prepared by copolymerizing the ammonium or volatile amine salt of an anionic monomer with the hydrophobic monomer.

The matrix polymer may be prepared by any suitable polymerization process. For instance, the polymer can be prepared by aqueous emulsion polymerization, such as the one described in EP-A-697423 or *U.S. Pat. Spec. No. 5,070,136.* The polymer can then be neutralized by the addition of an aqueous solution of ammonium hydroxide or a volatile amine.

In a typical polymerization process, the blend of hydrophobic monomer and anionic monomer is emulsified into an aqueous phase which contains a suitable amount of emulsifying agent. The emulsifying agent may be any commercially available emulsifying agent suitable for forming aqueous emulsion. These emulsifying agents will tend to be more soluble in the aqueous phase than in the water immiscible monomer phase and thus will tend to exhibit a high hydrophilic lipophilic balance (HLB). Emulsification of the monomer may be effected by known emulsification techniques, including subjecting the monomer/aqueous phase to vigorous stirring or shearing or alternatively passing the monomer/aqueous phase through a screen or mesh. Polymerization may then be effected by use of a suitable initiator system, for instance a UV initiator or thermal initiator. A suitable technique of initiating the polymerization would be to elevate the temperature of an aqueous emulsion of monomer to above 70 or 80°C and then add between 50 and 1000 ppm of ammonium persulphate by weight of monomer.

The matrix polymer has a molecular weight of up to 200,000 (determined by GPC using standard industrial parameters). Preferably the polymer has a molecular weight of below 50,000, for instance 2,000 to 20,000. According to a preferred embodiment, the optimum molecular weight for the matrix polymer is around 6,000 to 12,000.

A particularly preferred matrix polymer is a copolymer of styrene with ammonium acrylate. More preferably this polymer is used when the process employs a cross-linking agent, which is especially ammonium zirconium carbonate or zinc oxide.

In an alternative version of the process, the ionic monomer may be cationic or potentially cationic, for instance an ethylenically unsaturated amine. In this form of the invention the volatile counterionic component is a volatile acid component. The matrix polymer can be formed in an analogous way to the aforementioned anionic matrix polymer, except that the anionic monomer is replaced by a cationic or potentially cationic monomer. In the event that the polymer is prepared in the form of a copolymer of a free amine and hydrophobic monomer, it is neutralized by including a suitable volatile acid, for instance acetic acid or formic acid. Preferably the polymer is neutralized by a volatile carboxylic acid.

Suitable cationic or potentially cationic monomers include dialkyl aminoalkyl (meth) acrylates, dialkyl aminoalkyl (meth) acrylamides or allyl amines and other ethylenically unsaturated amines and their acid addition salts. Suitable dialkyl aminoalkyl (meth)acrylates include dimethyl aminomethyl acrylate, dimethyl aminomethyl methacrylate, 2-dimethylaminoethyl acrylate, dimethyl aminoethyl methacrylate, diethyl aminoethyl acrylate, diethyl aminoethyl methacrylate, dimethyl aminopropyl acrylate, dimethyl aminopropyl methacrylate, diethyl aminopropyl acrylate, diethyl aminopropyl methacrylate, dimethyl aminobutyl acrylate, dimethyl aminobutyl methacrylate, diethyl aminobutyl acrylate and diethyl aminobutyl methacrylate. Typical dialkyl aminoalkyl (meth) acrylamides include dimethyl aminomethyl acrylamide, dimethyl aminomethyl methacrylamide, dimethyl aminoethyl acrylamide, dimethyl aminoethyl methacrylamide, diethyl aminoethyl acrylamide, diethyl aminoethyl methacrylamide, dimethyl aminopropyl acrylamide, dimethyl aminopropyl methacrylamide, diethyl aminopropyl acrylamide, diethyl aminopropyl methacrylamide, dimethyl aminobutyl acrylamide, dimethyl aminobutyl methacrylate, diethyl aminobutyl acrylate and diethyl aminobutyl methacrylamide. Typical allyl amines include diallyl amine and triallyl amine.

The secondary particles comprise a hydrophobic polymer that has been formed from an ethylenically unsaturated hydrophobic monomer which is capable of forming a homopolymer of glass transition temperature in excess of 50°C and optionally other monomers, which hydrophobic polymer is different from the matrix polymer. The ethylenically unsaturated hydrophobic monomer may be any of the monomers defined above in respect of the second monomer used to form the matrix polymer. Preferably, the hydrophobic monomer is the same as the second monomer used to form the matrix polymer. Specific examples of said hydrophobic monomers include styrene, methyl (meth)acrylate, tertiary butyl methacrylate, phenyl methacrylate, cyclohexyl methacrylate and isobornyl methacrylate. Preferably the hydrophobic monomer is styrene.

The hydrophobic monomer may be polymerized alone or alternatively may optionally be polymerized with one or more other hydrophobic monomers as defined above. Other monomers that are not hydrophobic monomers capable of forming a homopolymer of glass transition temperature in excess of 50°C, may be included, such as hydrophobic monomers, for instance longer chain alkyl and esters of acrylic or methacrylic acid, such as 2-ethylhexyl acrylate or stearyl acrylate. In the event that such monomers are included, they should be present in an amount of no more than 20% by weight, based on to weight of monomers used for the secondary particles. Preferably, these monomers will be present in amount less than 10% by weight and more preferably less than 5% by weight.

Alternatively the other monomer may be a hydrophilic monomer. The hydrophilic monomer may be non-ionic, for instance acrylamide, or it can be ionic, for instance as defined in respect of the first monomer used to form the matrix polymer. Such monomers tend to be used in smaller proportions so that the polymer is hydrophobic. Where such monomers are included, they should be present in an amount of no more than 20% by weight based on to weight of monomers used for the secondary particles. Preferably, these monomers will be present in an amount less than 10% by weight and more preferably less than 5% by weight.

It is particularly preferred that the secondary particles comprise a hydrophobic polymer that has been formed entirely from one or more ethylenically unsaturated hydrophobic monomer(s) which is/are capable of forming a homopolymer of glass transition temperature in excess of 50°C. A particularly suitable hydrophobic polymer is a copolymer of styrene and methyl methacrylate or a homopolymer of styrene. The polymer of styrene with methyl methacrylate generally will comprise at least 40% by weight styrene and up to 60% by weight methyl methacrylate. Preferably, the copolymer will have a weight ratio of styrene to methyl methacrylate of between 50:50 to 95:5 and more preferably 60:40 to 80:20, particularly preferably 70:30 to 75:25.

The secondary particles will have an average particle size of below 1 µm, particularly below 750 nm. Preferably, the secondary particles will have an average particle size in the range between 50 and 500 nm. The secondary particles may be prepared by any conventional means, such as aqueous emulsion polymerization. Preferably, the particles are prepared by aqueous micro-emulsion polymerization according to any typical micro-emulsion polymerization process documented in the prior art, for instance as described in EP-A-531 005 or EP-A-449 450.

The secondary particles may be prepared by forming a micro-emulsion comprising a continuous aqueous phase (between 20 and 80% by weight), a dispersed oil phase comprising the monomer (between 10 and 30% by weight), and surfactant and/or stabilizer (between 10 and 70% by weight). Generally, the surfactant and/or stabilizer will exist predominantly in the aqueous phase. A preferred surfactant and/or stabilizer is an aqueous solution of the polymer used to form the polymeric matrix. A particularly preferred surfactant/stabilizer is a copolymer of ammonium acrylate with styrene, as defined above in relation to the matrix polymer.

Polymerization of the monomer in the micro-emulsion can be effected by a suitable initiation system, for instance a UV initiator or thermal initiator. A suitable technique of initiating the polymerization is, for instance, to elevate the temperature of the aqueous emulsion of monomer to above 70 or 80°C and then to add between 50 and 1000 ppm of ammonium persulphate or an azo compound such as azodiisobutyronitrile by weight of monomer. Alternatively, a peroxide, e.g. a room-temperature curing peroxide, or a photo-initiator may be used. It may be preferred that polymerization is carried out at about room temperature, e.g. with a photoinitiator.

The secondary particles comprise a polymer that has a molecular weight of up to 2,000,000 (determined by GPC using the standard industrial parameters). Preferably the polymer has a molecular weight of below 500,000, for instance 5,000 to 300,000. The optimum molecular weight for the polymeric secondary particles is between 100,000 and 200,000.

It is preferred that the secondary particles have a core shell configuration in which the core comprises the hydrophobic polymer surrounded by a polymeric shell. More preferably the secondary particles comprise a core comprising the hydrophobic polymer and a shell comprising the matrix polymer. It is particularly preferable that the shell of matrix polymer is formed around the core of hydrophobic polymer and during polymerization.

The particles according to the process of the invention comprise a colourant. They may additionally comprise further active ingredients, for instance UV absorbers, UV reflectors, flame retardants or active dye tracer materials.

The particles entrap one or more colourants, for instance a dye, pigment or lake. Suitable colourants include any organic or inorganic pigments or colourants approved for use in cosmetics by CTFA and the FDA such as lakes, iron oxides, titanium dioxide, iron sulphides or other conventional pigments used in cosmetic formulations.

Examples of suitable pigments include inorganic pigments such as carbon black, D&C Red 7, calcium lake, D&C Red 30, talc lake, D&C Red 6, barium lake, russet iron oxide, yellow iron oxide, brown iron oxide, talc, kaolin, mica, mica titanium, red iron oxide, magnesium silicate and titanium oxide; and organic pigments such as Red No. 202, Red No. 204, Red No. 205, Red No. 206, Red No. 219, Red No. 228, Red No. 404, Yellow No. 205, Yellow No. 401, Orange No. 401 and Blue No. 404. Examples of vat dyes are Red No. 226, Blue No. 204 and Blue No. 201. Examples of lake dyes include various acid dyes which are laked with aluminum, calcium or barium.

In one embodiment the colourant is an aqueous solution of a water-soluble dye. Such dyes may include FD&C Blue No. 11, FD&C Blue No. 12, FD&C Green No. 13, FD&C Red No. 13, FD&C Red No. 140, FD&C Yellow No. 15, FD&C Yellow No. 16, D&C Blue No. 14, D&C Blue No. 19; D&C Green No. 15, D&C Green No. 16, D&C Green No. 18, D&C Orange No. 14, D&C Orange No. 15, D&C Orange No. 110, D&C Orange No. 111, D&C Orange No. 117, FD&C Red No. 14, D&C Red No. 16, D&C Red No. 17, D&C Red No. 18, D&C Red No. 19, D&C Red No. 117, D&C Red No. 119, D&C Red No. 121, D&C Red No. 122, D&C Red No. 127, D&C Red No. 128, D&C Red No. 130, D&C Red No. 131, D&C Red No. 134, D&C Red No. 139, FD&C Red No. 140, D&C Violet No. 12, D&C Yellow No. 17, Ext. D&C Yellow No. 17, D&C Yellow No. 18, D&C Yellow No. 111, D&C Brown No. 11, Ext. D&C Violet No. 12, D&C Blue No. 16 and D&C Yellow No. 110.

The above dyes are well known, commercially available materials, with their chemical structure being described, e.g., in *21* C. *F. R. Part 74* (as revised April 1, 1988) and in the CTFA Cosmetic Ingredient Handbook 1988, published by the Cosmetics, Toiletry and Fragrances Association, Inc.*.*

The certified dyes can be water-soluble or, preferably, lakes thereof. Lakes are organic pigments prepared by precipitating a soluble dye on a reactive or absorbent stratum, which is an essential part of the pigment containing composition. Most lakes are aluminum, barium or calcium derived. These insoluble pigments are used mostly in makeup products, either powders or liquids, when a temporary colour is desired that does not stain the skin, as oil-soluble dyes tend to do. The lakes are used in these products along with inorganic colours, such as iron oxide, zinc oxide and titanium dioxide.

The colourant can also be a substance that is a dormant colourant, for instance a colour former that exhibits a colour on exposure to a suitable trigger mechanism, for instance heat or irradiation. Such entrapped colour formers can be coated onto or incorporated into suitable substrates and then treated to exhibit the colour. The colour former can still be activated even though it is entrapped within the polymer particle.

The following tables list currently available dyes and colourants approved for use in food, drugs and/or cosmetics. The selected colourant for use herein is preferably selected from the following exemplary lists.

**TABLE 1 - Dyes certified for use in foods, drugs, cosmetics (FDC colours)**

| | | |
|---|---|---|
| FD&C Blue No. 1 | FD&C Green No. 3 | FD&C Red No. 4 |
| FD&C Red No. 40 | FD&C Yellow No. 5 | FD&C Yellow No. 6 |

**TABLE 2 - Dyes certified for topically applied drugs and cosmetics**

| Ext. DC Violet #2 | Ext. D&C Yellow No. 7 | Ext. D&C Violet No. 2 |
|---|---|---|
| D&C Brown No. 1 | FD&C Red No. 4 | D&C Red No. 17 |
| D&C Red No. 31 | D&C Red No. 34 | D&C Red No. 39 |
| D&C Violet No. 2 | D&C Blue No. 4 | D&C Green No. 6 |
| D&C Green No. 8 | D&C Yellow No. 7 | D&C Yellow No. 8 |
| D&C Yellow No. 11 | D&C Orange No. 4 | D&C Orange No. 10 |
| D&C Orange No. 11 | | |

**TABLE 3 - Dyes certified for drugs and foods only**

| | | |
|---|---|---|
| D&C Blue No. 4 | D&C Brown No. 1 | D&C Green No. 5 |
| D&C Green No. 6 | D&C Green No. 8 | D&C Orange No. 4 |
| D&C Orange No. 5 | D&C Orange No. 10 | D&C Orange No. 11 |
| D&C Red No. 6 | D&C Red No. 7 | D&C Red No. 17 |
| D&C Red No. 21 | D&C Red No. 22 | D&C Red No. 27 |
| D&C Red No. 28 | D&C Red No. 30 | D&C Red No. 31 |
| D&C Red No. 33 | D&C Red No. 34 | D&C Red No. 36 |
| D&C Violet No. 2 | D&C Yellow No. 7 | D&C Yellow No. 8 |
| D&C Yellow No. 10 | D&C Yellow No. 11 | |

Some colour additives are exempt from certification and permanently listed for cosmetic use, including aluminum powder, annatto, bismuth oxychloride, bronze powder, caramel, carmine, beta-carotene, chromium hydroxide green, chromium oxide green copper (metallic powder), dihydroxyacetone, disodium EDTA-copper, ferric ammonium ferrocyanide, ferric ferrocyanide, guanine (pearl essence), guaiazulene (azulene), iron oxides, luminescent zinc sulphide, manganese violet, mica, pyrophyllite, silver (for colouring fingernail polish), titanium dioxide, ultramarines (blue, green, pink, red & violet), and zinc oxide.

The subsequent dehydration step can be achieved by any convenient means. Generally this will require elevated temperatures, for instance temperatures of 150°C or higher. Although it may be possible to use much higher temperatures e.g. up to 250°C, it is generally preferred to use temperatures of below 220°C. The dehydration can be achieved by the spray drying process described in *WO 97*/*34945.*

The dehydration step removes water from the aqueous solution of matrix polymer and also the volatile counterion component, resulting in a dry polymer matrix, which is insoluble and non-swellable in water, containing therein the colourant, which is distributed throughout the polymeric matrix.

Polymeric particles are obtained by the spray-drying process, wherein at least 90% by weight are of a size below 250 µm, preferably 100 µm. According to a preferred embodiment, at least 90% of the granules are of a particle size below 50 µm. The encapsulated colourant microspheres having average diameter sizes of 0.1 - 50 µm are preferred, for example 1-40 µm and especially 1 to 30 µm.

Preferred granules have a size of at least 90% in the range from 50 to 250 µm.

The granules of the desired particle size can be produced by subjecting the aqueous emulsion described above to conventional spray-drying conditions using a conventional spray drier, with the particle size being controlled in a known manner by appropriate selection of spray-drying orifices, rate of pumping through the orifices and the rate of drying (temperature and drier) dimensions of the spray-dried material.

Encapsulated colourant microspheres having average diameters of 0.1 to 60 µm are preferred, for example 1 to 40 and especially 1 to 30 µm.

Depending on the intended use, the preferred average diameters will vary. For example, a liquid facial cosmetic formulation comprises at least 2 encapsulated colourants and has preferred particle sizes of between 10 and 30 µm Another embodiment may be a lipstick formulation comprising at least 2 encapsulated colourants having preferred particle sizes of between 1 and 10 µm.

Applying a personal care or cosmetic formulation composition comprising (micro)encapsulated colourants obtained by the process of this invention produces desirable effects upon application. Notably, the compositions containing a blend of at least 2 microencapsulated colourants having unique and distinct colours, particularly a blend of more than one primary colour, are effective means for producing natural, textured skin tone effects. The primary colours are understood to mean red, yellow and blue. An additional advantageous feature of the encapsulated colourants is the elimination of milling or grinding often encountered with non-encapsulated colourants.

The personal care or cosmetic compositions comprise from 0.1 to 70% by weight, for example from 1 to 50% by weight, and especially from 5 to 35% by weight based on the total weight of the composition, of at least 1, preferably 2, encapsulated colourants obtained by the process of the invention, as well as a cosmetically tolerable carrier or adjuvant. While water is cosmetically tolerable, and in most instances will also be present, the phrase "a cosmetically tolerable carrier or adjuvant" is intended to refer to at least one substance other than water that is customarily employed in personal care or cosmetic compositions.

The personal care or cosmetic preparation may be formulated as a water-in-oil or oil-in-water emulsion, as a vesicular dispersion of an ionic or non-ionic amphiphilic lipid, as a gel, or a solid stick. Preferably the cosmetic preparation is in the form of a liquid.

As a water-in-oil or oil-in-water emulsion, the personal care or cosmetic preparation preferably contains from 5 to 50 % of an oily phase, from 5 to 20 % of an emulsifier and from 30 to 90 % water. The oily phase may contain any oil suitable for cosmetic formulations, e.g. one or more hydrocarbon oils, a wax, natural oil, silicone oil, a fatty acid ester or a fatty alcohol.

Cosmetic liquids may include minor amounts, for example up to 10 weight percent of mono- or polyols such as ethanol, isopropanol, propylene glycol, hexylene glycol, glycerol or sorbitol.

Cosmetic formulations may be contained in a wide variety of cosmetic preparations. Especially the following preparations, for example, come into consideration:
- skin-care preparations, e.g. skin emulsions, multi-emulsions or skin oils and body powders;
- cosmetic personal care preparations, e.g. facial make-up in the form of lipsticks, lip gloss, eye shadow, liquid make-up, day creams or powders, facial lotions, creams and powders (loose or pressed); and
- light-protective preparations, such as sun tan lotions, creams and oils, sun blocks and pretanning preparations.

The following examples illustrate the invention, but do not limit the scope thereof:

### Examples

### Particle Size Analysis

The mean particles sizes of polymer particles are determined with a Sympatec HELOS particle size analyser set up with a Quixcel dispersion system. The equipment is available from Sympatec GmbH.

### Example 1

### 1.1 Preparation of micro-emulsion polymer

To a 1 litre resin pot fitted with mechanical stirrer, condenser, nitrogen inlet, temperature probe and feed inlets are placed 154.85 g water and 483.9 g of a 32% solution of an ammonium salt of a low molecular weight styrene acrylic copolymer (65/35 wt.-% monomers ratio, molecular weight 6000). The contents are heated to 85°C and degassed with nitrogen for 30 minutes. A monomer phase is prepared by mixing 245.0 g styrene with 105.0 g methyl methacrylate. An initiator feed is prepared by dissolving 1.97 g ammonium persulphate in 63.7 g water. When the reactor is at temperature and degassed, 0.66 g ammonium persulphate are added to the reactor. After 2 minutes the monomer and initiator feeds are started appropriate to a 3 and 4 hour feed respectively. The reactor contents are maintained at 85°C throughout the feeds. After completion of the feeds, the reactor contents are held for a further 1 hour at 85°C and then cooled down to 25°C. The resulting aqueous product is a micro-emulsion polymer at pH 8.3 and with a Brookfield RVT viscosity of 700 [mPas]. The 46% polymer microemulsion contains 32% by weight of styrene-methyl methacrylate copolymer (70/30 wt.-% monomer ratio, molecular weight 200,000) micro-emulsion stabilised with 14 wt.-% styrene-acrylic acid (65/35 wt.% monomers ratio, molecular weight 6000). The mean particle size of the dispersed latex polymer of styrene-methyl methacrylate copolymer is 195 nm.

### 1.2 This example illustrates the preparation of yellow coloured polymer particles containing of 10% by weight of yellow pigment.

An aqueous feed is prepared by diluting 155.4 g of 46% polymer micro-emulsion prepared according to Example 1.1 with 100.0 g of deionised water followed by addition of a 1.0 g of a de-foamer Burst 5470 (ex-Ciba). This diluted mixture is placed under an overhead homogeniser (Silverson L4R) and then 10.0 g of Yellow #5 Al lake powder (ex-Kingfisher) and 20.0 g of zinc oxide (ex-Norkem Chemicals) added under high shear mixing. The resulting coloured mixture is homogenised for total time of 15 minutes to form a uniform smooth dispersion of pigments in the aqueous micro-emulsion polymer.

The dispersion is spray dried at an inlet temperature of 180°C at a feed rate of 3ml/min using a laboratory spray dryer (Buchi Model B191). The final product is a free flowing yellow microbead having a mean particle size diameter of 25 µ. The matrix polymer is zinc oxide crosslinked styrene-acrylic acid copolymer wherein the secondary polymer particles of styrene-methyl methacrylate of around 200 nanometer size are distributed throughout the matrix.

### Example 2

A red coloured micro-bead is prepared in the same manner as described in Example 1 except that 20.0 g of Red #36 pigment (ex-Ciba) is used instead of the Yellow #5 Al lake powder.

### Example 3

A blue coloured micro-bead is prepared in the same manner as described in Example 1 except that 20 g of Blue Al lake (ex-Kingfisher) is used instead of the Yellow #5 Al lake powder.

### Example 4

This example demonstrates the viability of the process on a pilot scale spray dryer. A diluted polymer solution is prepared by mixing 309.2 kg of 46% of polymer micro-emulsion having the same composition as described in Example 1 with 129.2 kg of water and 2.0 kg of Burst 5470 defoamer. To this diluted polymer solution is added 19.9 kg FDC yellow 5 Al lake and 39.7 kg zinc oxide. The resulting mixture is placed under a high shear mixer (Drais dissolver) and mixed for 30 minutes to form a yellowed coloured aqueous dispersion. A pilot plant spray dryer DT3 equipped with a 2 two-substance nozzles was used for drying the yellowed coloured dispersion. The aqueous dispersion iss pumped at a rate of 200 kg/hour rate at 5 bar air pressure and outlet temperature of 110°C. The aqueous feed is spray dried to yield a yellowed powder product having a mean microbead particle sizes of 20 µm with a water content of 2.3% as measured by Karl Fischer method.

## Claims

1. A process of preparing polymer particles which comprise an effective amount of at least one colourant, a polymer matrix and secondary particles distributed within the polymer matrix,
wherein the polymer matrix is formed from a blend of monomers comprising a first monomer, which is an ethylenically unsaturated ionic monomer, and a second monomer, which is an ethylenically unsaturated hydrophobic monomer and which is capable of forming a homopolymer of glass transition temperature above 50°C,
wherein the secondary particles comprise a hydrophobic polymer which is formed from an ethylenically unsaturated hydrophobic monomer which is capable of forming a polymer of glass transition temperature above 50°C as determined by differential scanning calorimetry (DSC) and optionally other monomers, and wherein the hydrophobic polymer is different from the polymeric matrix,
which process essentially consists of the steps:
A) Preparing an emulsion of a polymer formed from a monomer blend in an aqueous phase which comprises the first and second monomers, forming the secondary particles in the aqueous phase or combining the secondary particles with the aqueous phase;
B) Adding to the aqueous phase an effective colouring amount of at least one colourant;
C) Dispersing or dissolving the colourant in the aqueous phase; and
D) Subjecting the emulsion to dehydration in a spray-drying unit.

2. A process according to claim 1, wherein the polymer matrix is formed from the ammonium salt of a low molecular weight styrene acrylic copolymer.

3. A process according to claim 1, wherein the secondary particles are formed from a styrene-methyl methacrylate copolymer.

4. A process according to claim 1, which comprising dispersing the colourant selected from the group consisting of at least one colourant selected from the group consisting of pigments, dyes or lakes.

5. A process according to claim 1, which essentially consists of the steps:
A) Preparing an emulsion of the ammonium salt of a low molecular weight styrene acrylic copolymer, forming in the aqueous phase the secondary particles from a styrene-methyl methacrylate copolymer;
B) Adding to the aqueous phase an effective colouring amount of at least one colourant;
C) Dispersing or dissolving the colourant in the aqueous phase; and
D) Subjecting the emulsion to dehydration in a spray-drying unit.

6. A process according to claim 1, wherein the polymer particles have an average particle size below 100 µm.

7. A process according to claim 1, wherein the secondary particles have an average particle size below 750 nm.

8. A process according to claim 1, wherein two different colourants are dispersed in the aqueous phase.

9. A process for the preparation of personal care or cosmetic compositions, which comprises further processing to personal care or cosmetic compositions the polymer particles obtainable by the process according to claim 1.

## Patentansprüche

1. Verfahren zur Herstellung von Polymerpartikeln, die eine wirksame Menge von mindestens einem Farbmittel, eine Polymermatrix und in der Polymermatrix verteilte Sekundärpartikel umfassen,
wobei die Polymermatrix aus einem Monomer-Blend, umfassend ein erstes Monomer, bei dem es sich um ethylenisch ungesättigtes ionisches Monomer handelt, und ein zweites Monomer, bei dem es sich um ein ethylenisch ungesättigtes hydrophobes Monomer handelt und das fähig ist, ein Homopolymer mit einer Glasumwandlungstemperatur von über 50°C zu bilden, gebildet ist,
wobei die Sekundärpartikel ein hydrophobes Polymer umfassen, das aus einem ethylenisch ungesättigten hydrophoben Monomer, das fähig ist, ein Polymer mit einer Glasumwandlungstemperatur von über 50°C gemäß Bestimmung mittels Differenzialscanningkalorimetrie (DSC) zu bilden und gegebenenfalls anderen Monomeren gebildet ist und worin sich das hydrophobe Polymer von der Polymermatrix unterscheidet,
wobei das Verfahren im Wesentlichen aus den folgenden Schritten besteht:
A) Herstellen einer Emulsion eines Polymers aus einem Monomer-Blend in einer wässrigen Phase, die das erste und das zweite Monomer umfasst, Bilden der Sekundärpartikel in der wässrigen Phase oder Kombinieren der Sekundärpartikel mit der wässrigen Phase;
B) Versetzten der wässrigen Phase mit einer farbwirksamen Menge von mindestens einem Farbmittel;
C) Dispergieren oder Lösen des Farbmittels in der wassrigen Phase; und
D) Entwässern der Emulsion in einer Sprühtrocknereinheit.

2. Verfahren nach Anspruch 1, wobei die Polymermatrix aus dem Ammoniumsalz eines niedermolekularen Styrol-Acryl-Copolymers gebildet wird.

3. Verfahren nach Anspruch 1, wobei die Sekundärpartikel aus einem Styrol-Methylmethacrylat-Copolymer gebildet werden.

4. Verfahren nach Anspruch 1, bei dem das aus der Gruppe bestehend aus mindestens einem Farbmittel, ausgewählt aus der Gruppe bestehend aus Pigmenten, Farbstoffen oder Farblacken, ausgewählte Farbmittel dispergiert wird.

5. Verfahren nach Anspruch 1, das im Wesentlichen aus den folgenden Schritten besteht:
A) Herstellen einer Emulsion des Ammoniumsalzes eines niedermolekularen Styrol-Acryl-Copolymers, Bilden der Sekundärpartikel aus einem Styrol-Methylmethacrylat-Copolymer in der wässrigen Phase;
B) Versetzten der wässrigen Phase mit einer farbwirksamen Menge von mindestens einem Farbmittel;
C) Dispergieren oder Lösen des Farbmittels in der wässrigen Phase; und
D) Entwässern der Emulsion in einer Sprühtrocknereinheit.

6. Verfahren nach Anspruch 1, wobei die Polymerpartikel eine durchschnittliche Partikelgröße unter 100 µm aufweiten.

7. Verfahren nach Anspruch 1, wobei die Sekundärpartikel eine durchschnittliche Partikelgröße von unterhalb 750 nm aufweiten.

8. Verfahren nach Anspruch 1, wobei zwei unterschiedliche Farbmittel in der wässrigen Phase dispergiert worden.

9. Verfahren für die Herstellung von Körperpflege - oder Kosmetikzusammensetzungen, bei dem die nach dem Verfahren nach Anspruch 1 erhältlichen Polymerpartikel zu Körperpflege- oder Kosmetikzusammensetzungen weiter verarbeitet werden.

## Revendications

1. Procédé de préparation de particules polymères qui comprennent une quantité efficace d'au moins un colorant, une matrice polymère et des particules secondaires distribuées à l'intérieur de la matrice polymère,
dans lequel la matrice polymère est constituée d'un mélange de monomères comprenant un premier monomère, qui est un monomère ionique éthyléniquement insaturé, et un deuxième monomère, qui est un monomètre hydrophobe éthyléniquement insaturé et qui est susceptible de former un homopolymère avec une température de transition vitreuse supérieure à 50 °C,
dans lequel les particules secondaires comprennent un polymère hydrophobe qui est constitué d'un monomètre hydrophobe éthyléniquement insaturé qui est susceptible de former un polymère avec une température de transition vitreuse supérieure à 50 °C comme déterminé par calorimétrie différentielle à balayage (DSC) et éventuellement d'autres monomères, et dans lequel le polymère hydrophobe est différent de la matrice polymère,
lequel procécé est essentiellement composé des étapes suivantes :
A) préparer une émulsion d'un polymère constitué d'un mélange de monomères dans une phase aqueuse qui comprend le premier et le deuxième monomère, former les particules secondaire dans la phase aqueuse ou combiner les particules secondaires avec la phase aqueuse ;
B) ajouter à la phase aqueuse une quantité colorante efficace d'au moins un colorant ;
C) disperser ou dissoudre le colorant dans la phase aqueuse ; et
D) soumettre l'émulsion à une déshydratation dans une unité de séchage par atomisation.

2. Procédé selon la revendication 1, dans lequel la matrice polymère est constituée du sel d'ammonium d'un copolymère styrène-acrylique de bas poids moléculaire.

3. Procédé selon la revendication 1, dans lequel les particules secondaires sont constituées d'un copolymère styrène-méthacrylate de méthyle.

4. Procédé selon la revendication 1, qui comprend la dispersion du colorant choisi dans le groupe constitué par au moins un colorant choisi dans le groupe constitué par les pigments, les teintures et les laques.

5. Procédé selon la revendication 1, qui est essentiellement composé des étapes suivantes :
A) préparer une émulsion du sel d'ammonium d'un copolymère styrène-acrylique de bas poins moléculaire, former dans la phase aqueuse les particules secondaires à partir d'un copolymère styrène-méthacrylate de méthyle ;
B) ajouter à la phase aqueuse une quantité colorante efficace d'au moins un colorant ;
C) disperser ou dissoudre le colorant dans la phase aqueuse ; et
D) soumettre l'émulsion à une déshydratation dans une unité de séchage par atomisation.

6. Procédé selon la revendication 1, dans lequel les particules polymères ont une taille moyenne de particules inférieure à 100 µm.

7. Procédé selon la revendication 1, dans lequel les particules secondaires ont une taille moyenne de particules inférieure à 750 nm.

8. Procédé selon la revendication 1, dans lequel deux colorants différents sont dispersés dans la phase aqueuse.

9. Procédé de préparation de compositions de soins personnels ou cosmétiques, qui comprend en outre la transformation en compositions de soins personnels ou cosmétiques des particules polymères pouvant être obtenues par le procédé selon la revendication 1.
